# EUROPEAN PATENT APPLICATION

(11) **EP 2 644 087 A1**
(43) Date of publication of application: **02.10.2013**
(21) Application number: 13000613.3
(22) Date of filing: 06.02.2013
(51) Int. Cl.: A61B 5/00, G06F 19/00, A61B 5/11, G06F 11/16

(54) **Sleep management system and sleep monitor**

(30) Priority: 30.03.2012 JP 2012080626
(71) Applicant: Tanita Corporation, Tokyo 174 (JP)
(72) Inventor: Sato, Tomio, Tokyo, 174-8630 (JP); Shiota, Takayuki, Tokyo, 174-8630 (JP)
(74) Representative: Müller-Boré & Partner Patentanwälte

(57) **Abstract**

A sleep measurement system comprises a sleep monitor (10) for measuring data relating to a user's sleep, an external terminal (50) for displaying a state of said user's sleep, a first server (30) for storing first data received from said sleep monitor (10) and providing the first data to said external terminal (50), and a second server for analyzing said user's sleep on the basis of second data received from said sleep monitor (10) and providing an analysis result to said external terminal (50). Said sleep monitor (10) processes the information in each epoch, sends a processing result of said biometric information in each epoch to said first server (30) as the first data, and sends said biometric data accumulated during said user's sleep and said processing result of said biometric information in each said epoch to said second server (40) as said second data.

## Description

### TECHNICAL FIELD

The present invention relates to sleep management system and a sleep monitor.

### RELATED ART

Heretofore, a sleep monitor for measuring biometric information during user's sleep or performing some evaluation on the user's sleep state in a certain degree on the basis of the biometric information measurement has been provided (see patent publications 1 and 2 for example). This sleep monitor enables to confirm with specific numerical evidences, for example, how many hours the user could substantially sleep after going to bed and before waking up.

[Patent Publication 1] Japanese Patent Application Laid-Open Publication No. 2006-280686
[Patent Publication 2] Japanese Patent Application Laid-Open Publication No. 2008-301951

### SUMMARY OF THE INVENTION

Since a conventional sleep monitor does not have a network capability, it has not been considered, for example, to allow a server on the network to receive biometric information from the sleep monitor and provide service relating to sleep to an external terminal. In particular, as a service using biometric information relating to sleep, a framework for providing dissimilar services such as a service requiring highly timely updates to, for example, prevent a resident in a nursing home from nighttime wandering, and a service requiring a complicated sleep analysis to, for example, evaluate a sleep state of a driver of a transportation company on the day before driving.

Accordingly, in view of the circumstances as described above, an object of the present invention is to provide a sleep management system and a sleep monitor that can distinctly manage both of a service requiring highly timely updates and a service performing a sleep analysis on the basis of biometric information measured by a sleep monitor with a network capability.

In order to solve the various problems described above, a sleep management system according to the present invention comprises, a sleep monitor for measuring biometric information relating to user's sleep, an external terminal for displaying a state relating to said user's sleep, a first server for storing first data received from said sleep monitor and providing the first data to said external terminal, a second server for conducting sleep analysis on the basis of second data received from said sleep monitor and providing analysis result to said external terminal, wherein said sleep monitor processes said biometric information in each unit interval, sends said processing result of said biometric information in each unit interval to said first server as said first data, and sends said biometric data accumulated during said user's sleep and said processing result of said biometric information in each said unit interval to said second sever as said second data.

In a preferred embodiment, said sleep monitor may send said first data to said first server in every predetermined time period defining said unit interval.

In a preferred embodiment, at a certain time point of said user's sleep, said sleep monitor may send said second data accumulated up to said time point to said second server.

In a preferred embodiment, said biometric information data may contain data of a plurality of channels with different amplification factors.

In a preferred embodiment, said biometric information data may contain data of only one channel among data of a plurality of channels with different amplification factors.

In a preferred embodiment, said external terminal may be capable of selecting the data to be received from said first and second server for each said user.

In a preferred embodiment, said sleep monitor, in case of communication failure with at least one of said first and second servers, may notify said external terminal of the failure.

In a preferred embodiment, said sleep monitor, in case of communication failure with any one of said first and second servers, may send data supposed to be sent to the incommunicable server to the communicable server.

In a preferred embodiment, said sleep monitor, when said incommunicable server recovers from the failure, may send a predetermined notice to at least one of said recovered server and said communicable server so that said recovered server obtains the data supposed to be received during the communication failure from said communicable server.

In order to solve the various problems described above, a sleep monitor according to the present invention comprises, a sensor for measuring biometric information relating to user's sleep, a microcomputer for processing said biometric information in each unit interval, a storage unit for storing data of said biometric information and a processing result of said biometric information, and an external communication unit for sending said data of said biometric information and said processing result of said biometric data, wherein said external communication unit respectively sends first data containing said processing result of said biometric information in each said unit interval, and second data containing said biometric data accumulated during said user's sleep and said processing result of biometric data in said each unit interval to various external servers.

In a preferred embodiment, said external communication unit may send said first data to said first server in every predetermined time period defining said unit interval.

In a preferred embodiment, at a certain time point of said user's sleep, said external communication unit may send said second data accumulated up to said time point to said second server.

In a preferred embodiment, said biometric information data may contain data of a plurality of channels with different amplification factors.

In a preferred embodiment, said biometric data may contain data of only one channel among data of a plurality of channels with different amplification factors.

In a preferred embodiment, said external communication unit, in case of communication failure with at least one of said first and second servers, may send a notice of the failure to said external terminal.

In a preferred embodiment, said external communication unit, in case of communication failure with any one of said first and second servers, may send data supposed to be sent to the incommunicable server to the communicable server.

In a preferred embodiment, said external communication unit, when said incommunicable server recovers from the failure, may send a predetermined notice to at least one of said recovered server and said communicable servers so that said recovered server obtains the data supposed to be received during the communication failure from said communicable server.

According to the sleep management system and the sleep monitor of the present invention, it is possible to distinctly manage both of the service requiring highly timely updates and the service performing a sleep analysis on the basis of biometric information measured by a sleep monitor with a network capability.

### BRIEF DESCRIPTION OF DRAWINGS

FIG. 1 illustrates a configuration of an embodiment of a sleep management system according to the present invention.
FIG. 2 illustrates a first example of an application of the sleep management system according to the present invention.
FIG. 3 illustrates a second example of an application of the sleep management system according to the present invention.
FIG. 4 illustrates a modified example of the sleep management system according to the present invention.

### DESCRIPTION OF EMBODIMENTS

Hereinafter, an embodiment of the present invention is described in detail with reference to the drawings.

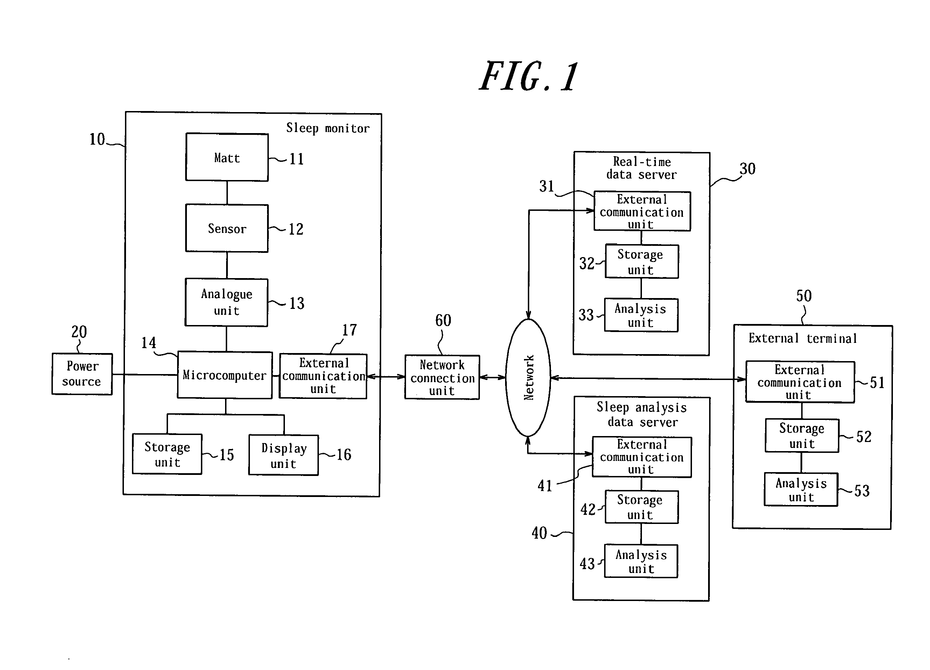

FIG. 1 illustrates a configuration of an embodiment of a sleep management system according to the present invention. The sleep management system has a sleep monitor 10 for measuring biometric data relating to user's sleep, a power source 20 for providing electricity power to the sleep monitor 10, a real-time data server 30 (first server) for providing a service requiring highly timely updates, a sleep analysis data server 40 (second server) for providing a service requiring a sleep analysis, an external terminal 50 for displaying a state of the user's sleep, and a network connection unit 60 for connecting sleep monitor 10 to a network. The sleep monitor 10 and the external terminal 50 are connected to the real-time data server 30 and the sleep analysis data server 40 via a network such as the internet. It is noted that although only one sleep monitor 10 is depicted in FIG. 1 for a purpose of illustration, more than one sleep monitors 10 can be connected to the real-time data server 30 or the sleep analysis data server 40. A plurality of external terminals 50 may be used as well.

The sleep monitor 10 has a mat 11, a sensor 12, an analog circuit 13, a microcomputer 14, a storage unit 15, a display unit 16 and an external communicate unit 17.

The mat 11 is a rug for resting the body of the sleeping user. For example, the mat 11 is used in a condition that bedding such as futon or mattress for the user lying down is placed on the mat 11, or in a state that the user directly lies on the upper surface of the mat 11.

The sensor 12 detects biometric signals of the user lying on the mat 11. The sensor 12 is equipped with, for example, a microphone such as a condenser microphone to measure a pressure variation of the mat 11 and detect the biometric signals such as respiratory rates and heart rates and postural changes or body movement of the user in a supine. Hereinafter, the biometric signals and the body movement detected by the sensor 12 are referred to as biometric information relating to sleep. In addition, biometric information data means raw data of the biometric information detected in a time series manner, such as waveform data.

An analog circuit 13 performs A/D conversion of the biometric information detected by the sensor 12 and outputs the biometric information to the microcomputer 14. It is noted that the analog circuit 13 may extract data in a predetermined range (e.g. a predetermined frequency) from the biometric information data by using a filter and amplify the extracted biometric information data by using an amplifier.

The microcomputer 14 is fed by an electric power source 20 and processes the biometric information detected by the sensor 12. In particular, the microcomputer 14 processes the biometric information in each unit interval (hereinafter referred to as "epoch") with predetermined time (e.g. 30 seconds) being used as a unit. For example, the microcomputer 14 may collect heart rates and respiratory rate in every epoch. The microcomputer 14 may calculate the standard deviation of the biometric information data in every epoch. Further, the microcomputer 14 may determine the user's in-bed/out-of-bed state in every epoch based on the fluctuation of the biometric information data such as respiration data. Further, the microcomputer 14 may determine the user's sleep/wake state in every epoch based on the fluctuation of the biometric information data such as body movement data. Further, microcomputer 14 may determine whether the user is apnea or not in every epoch based on the biometric information such as respiration data. It is noted that the microcomputer 14 may process the biometric information in every epoch in every predetermined time period defining said unit interval.

The storage unit 15 stores the biometric information data and the processing result obtained from the microcomputer 14 in every epoch.

The display unit 16 displays the operation state of sleep unit 10 and the processing result from the microcomputer 14.

The external communication unit 17 is a communication interface connected to the network via the network connection unit 60, and communicates with the real-time data server 30 and the sleep analysis data server 40. It is noted that the external communication unit 17 may also be directly connected to the network not via the network connection unit 60 to communicate with the real-time data server 30 and the sleep analysis data server 40.

The external communication unit 17 sends data (a first data) including the processing result of the biometric information in every epoch to the real-time data server 30. The external communication unit 17 preferably sends data including the processing result of the biometric information to the real-time data server 30 in each predetermined time period defining the epoch. For example, the external communication unit 17, in each predetermined time period defining the epoch, transmits processing results such as heart rates in the current epoch, respiratory rates in the current epoch, the standard deviation of the data in the current epoch, the determination result of in-bed/out-of-bed state in the current epoch and the determination results of sleep-wake state in the current epoch. The size of the data sent by the external communication unit 17 to the real-time data server 30 may be as small as, for example, about 100 bytes.

The external communication unit 17 also sends data of the biological information data accumulated during user's sleep (e.g. during in-bed time) and data including the processing result of the biometric information in every epoch (second data) to the sleep analysis data server 40. For example, when the user wakes up, the external communication unit 17 transmits data of the biometric information accumulated in the storage unit 15 from the beginning to the end of the user's sleep and the processing results such as heart rates in every epoch, respiratory rates in every epoch, the standard deviation in every epoch, the determination result of in-bed/out-of-bed state in every epoch and the determination result of sleep-awake state in every epoch. The size of the data sent by the external communication unit 17 to the sleep analysis data server 40 is relatively large and may be 800 kilobytes to 2.5 megabytes.

It is noted that the data transmission from the external communication unit 17 to the sleep analysis data server 40 is not limited at the time when the user wakes up, but the external communication unit 17 may send, at any time during the user's sleep (e.g. in every predetermined time period or at the time when any communication is not performed on the other sleep monitors 10), the data of the biometric information accumulated in the storage unit 15 until then and the processing results of the biometric information in every epoch to the sleep analysis data server 40. Further, the external communication unit 17 may also transmit the data to the sleep analysis data server 40 a predetermined time period after the user wakes up. In this way, by dispersing the timings of the data transmissions, a disadvantage that the data transmissions concentrate in the early morning can be solved, which reduces the load of the sleep analysis data server 40.

Furthermore, in case of communication failure, the external communication unit 17 resends the failed data to the real-time data server 30 and the sleep analysis data server 40 in the next epoch or after finishing the measurement. For example, when communication failure occurs in a certain epoch, the external communicate unit 17 may send the failed data to the real-time data server 30 in the next epoch. In addition, when communication failure occurs in the data transmission to the sleep analysis data server 40 upon the user getting up, the external communicate unit 17 may resend the data to the sleep analysis data server 40 after waiting for a predetermined time period.

In addition, in case of communication failure, the external communication unit 17 may identify the location (the server or the network connection unit) with the malfunction or failure and notify the external terminal 50 of it through the communicable server. In particular, when the communication failure occurs in at least one of the real-time data server 30 and the sleep analysis data server 40, the external communication unit 17 may notify the external terminal 50 of the failure. For example, when the external communication unit 17 cannot send data to the real-time data server 30 for a certain period of time, the external communication unit 17 may confirm whether it can communicate with each of the network connection unit 60 and the sleep analysis data server 40. If the external communication unit 17 can communicate with both of the network connection unit 60 and the sleep analysis data server 40, the external communicate unit 17 determines that the real-time data server 30 is incommunicable due to a malfunction, and notifies the sleep analysis data server 40 of it. This allows the sleep analysis data server 40 to notify the external terminal 50 that the real-time data server 30 is incommunicable due to malfunction or failure. It is noted that, when the sleep analysis data server 40 is incommunicable due to malfunction or failure, the external communication unit 17 may take the similar processing and notify the external terminal 50 of the failure through the real-time data server 30.

In addition, when the external communication unit 17 determines that any one of the real-time data server 30 and the sleep analysis data server 40 is incommunicable, the external communication unit 17 may send the data which is supposed to be sent to the incommunicable server to the communicable server. This allows the communicable server to store the data which is supposed to be sent to the incommunicable server in the storage unit of the communicable server. In other words, the external terminal 50 can access to the communicable server to obtain the information relating to the user's sleep which is supposed to be obtained from the incommunicable server. In addition, when the incommunicable server recovers to a normal operation, the recovered server can obtain the data which is supposed to be received during the communication failure from the communicable server. It is noted that, when the incommunicable server recovers to a normal operation, the external communication unit 17 may send a predetermined notice to at least one of the recovered server and the communicable server so that the recovered server can obtain the data which is supposed to be received during the communication failure from the communicable server. This allows an appropriate data communication between the recovered server and the communicable server.

When the external communication unit 17 determines that both of the real-time data server 30 and the sleep analysis data server 40 are incommunicable, the external communication unit 17 may send the data which is supposed to be sent to both of the servers to the external terminal 50. This allows the external terminal 50 to directly obtain and analyze the state relating to the user's sleep. It is noted that, when the external communication unit 17 determines that any one of the real-time data server 30 and the sleep analysis data server 40 is incommunicable, the external communication unit 17 may send the data supposed to be sent to the incommunicable server to external terminal 50.

In addition, as the biometric information data sent from the external communication unit 17 to the sleep analysis data server 40, the data of a plurality of channels with different amplification factors may be transmitted. The biometric information data varies according to the internal environment changes of the sleep monitor 10 such as a change of water in mat 11 over time, a temperature change due to the installation environment of the mat 11 and instrumental error of outputs from the sensor 12, as well as the external changes of the sleep monitor 10 such as a body type and body weight of the user, the position where the body lays against the mat 11 and the thickness, hardness and the type (futon, mattress) of the bedding. Therefore, the analog circuit 13 generates data of a plurality of channels with different amplification factors for the data of the biometric information from sensor 12, and the external communication unit 17 sends the data of the plurality of channels to the sleep analysis data server 40. By using the data of the plurality of channels with different amplification factors, the sleep analysis data server 40 can analyze the factors of changes caused by the environments, and conduct sleep analysis independent from the measuring environment. It is noted that, as the data of the plurality of channels with various amplification factors, 4 (four) channels of data may be sent with the amplification factors being respectively set to 2 times, 8 times, 32 times and 195.2 times. Further, the external communication unit 17 may send only one channel of data suitable for the sleep analysis among the data of the plurality of channels with different amplification factors to the sleep analysis data server 40. This allows the amount of data sent to the sleep analysis data server 40 to be significantly reduced, and thus makes it possible to decrease the processing load of the sleep analysis data server 40. It is noted that the selection of a suitable channel for the sleep analysis can be performed by, for example, the microcomputer 14 analyzing the waveform of the data of each channel.

The real-time data server 30, which stores the data received from the sleep monitor 10 and provides the data to the external terminal 50, has an external communication unit 31 as an interface for communication, a storage unit 32 for storing the data received from the sleep monitor 10, and an analysis unit 33 for analyzing the data stored in the storage unit 32. In particular, the storage unit 32 stores the data received from the sleep monitor 10 for each epoch. In addition, the external communication unit 31 provides the data stored in the storage unit 32 to the external terminal 50 when accessed from the external terminal 50. This allows the real-time data server 30 to provide information such as heart rates in the current epoch, transitions of heart rates over the epochs, respiratory rates in the current epoch, transitions of respiratory rates over the epochs, standard deviation of the data in the current epoch, transitions of standard deviation over the epochs, determination of in-bed/out-of-bed in the current epoch, transitions of determination of in-bed/out-of-bed over the epochs, determination of sleep/wake in the current epoch, and transitions of determination of sleep/wake over the epochs to the external terminal 50.

In addition, in case of communication failure, the real-time data server 30 may identify the location (the sleep monitor or the network connection unit) with the malfunction or failure and notify the external terminal 50 of it. For example, when the real-time data server 30 receives no data from the sleep monitor 10 for a certain period of time, the real-time data server 30 may confirm whether it can communicate with each of the network connection unit 60 and the sleep monitor 10. For example, when the real-time data server 30 cannot confirm the communication with the network connection unit 60, the real-time data server 30 may notify the external terminal 50 of the malfunction of the network connection device 60. In addition, when the real-time data server 30 can confirm the communication with the network connection unit 60 but cannot confirm the communication with the sleep monitor 10, the real-time data server may notify the external terminal 50 of the malfunction of the sleep monitor 10.

Moreover, when the sleep analysis data server 40 is incommunicable and the data supposed to be sent to the sleep analysis data server 40 is transmitted from the sleep monitor 10, the real-time data server 30 may store the data transmitted from the sleep monitor 10 in the storage unit 32 on behalf of the sleep analysis data server 40. Further, when the sleep analysis data server 40 recovers to a normal operation, the real-time data server 30 may send the data stored in the storage unit 32 during the communication failure to the sleep analysis data server 40.

When the real-time data server 30 is incommunicable due to its own malfunction, the real-time data server 30 may obtain the data supposed to be received from the sleep monitor 10 during the communication failure from the sleep analysis data server 40 after recovering to a normal operation.

It is noted that the real-time data server 30 may send/receive the data during the communication failure to/from the sleep analysis data server 40 in accordance with the predetermined notification from the sleep monitor 10.

The sleep analysis data server 40, which conducts a sleep analysis based on the data received from the sleep monitor 10 and provides the analysis results to the external terminal 50, has an external communication unit 41 as an interface for communication, a storage unit 42 for storing the data received from the sleep monitor 10, and an analysis unit 43 for analyzing the data stored in the storage unit 42. In particular, the analysis unit 43 conducts the sleep analysis based on the data received from the sleep monitor 10, and stores the analysis results in the storage unit 42. For example, the analysis unit 43 may analyze total sleep time, in-bed time and out-of-bed time with respect to in-bed/out-of bed; roll-over number and roll-over period with respect to body movements; average heart rates, maximum heart rates and minimum heart rates with respect to heart rates; and sleep time, sleep-onset latency, out-of-bed latency, deep sleep latency, REM sleep latency, mid-arousal period, mid-arousal number, sleep efficiency, arousal occurrence period, light sleep occurrence period, REM sleep occurrence period, deep sleep occurrence period, arousal occurrence ratio, light sleep occurrence ratio, REM sleep occurrence ratio, deep sleep occurrence ratio, arousal occurrence number, light sleep occurrence number, REM sleep occurrence number, deep sleep occurrence number, sleep stage change number, sleep cycle, ratio of deep sleep in the first half and the second half of sleep and AHI (Apnea Hypopnea Index) with respect to the sleep/wake determination. In addition, the analysis unit 43 may utilize parameters indicating the state of sleep such as sleep-onset latency, deep sleep latency, deep sleep occurrence period, mid-arousal number, roll over number, and sleep efficiency to determine a sleep score (sleep index) indicating quality of sleep and the type of sleep, and to analyze the depth of sleep (sleep stage) in each epoch. The external communication unit 41 provides analysis results stored in the storage unit 42 to external terminal 50 when accessed by the external terminal 50. This enables the sleep analysis data server 40 to provide a variety of sleep analysis results with the external terminal 50. It is noted that any method known to those skilled in the art may be used to perform various analysis at the analysis unit 43. Although applicable methods are not detailed herein, it can be found Patent Documents 1 and 2, if necessary.

In addition, in case of communication failure, the sleep analysis data server 40 may identify the location (the sleep monitor or the network connection unit) with the malfunction and notify the external terminal 50 of it. For example, when the sleep analysis data server 40 receives no data from the sleep monitor 10 for a certain period of time, the sleep analysis data server may confirm whether it can communicate with each of the network connection unit 60 and the sleep monitor 10. For example, when the sleep analysis data server 40 cannot confirm the communication with the network connection unit 60, the sleep analysis data server 40 may notify the external terminal 50 of the malfunction of the network connection unit 60. In addition, when the sleep analysis data server 40 can confirm the communication with the network connection unit 60 but cannot confirm the communication with the sleep monitor 10, sleep analysis data server 40 may notify the external terminal 50 of the malfunction of the sleep monitor 10.

In addition, the sleep analysis data server 40 may store the data transmitted from the sleep monitor 10 in the storage unit 42 on behalf of the real time data server 30 when the real-time data server 30 is incommunicable and the sleep monitor 10 sends the data supposed to be transmitted to the real-time data server 30. Further, when the real-time data server 30 recovers to a normal operation, the sleep analysis data server 40 may send the data stored in the storage unit 42 during the communication failure to the real-time data server 30.

When the sleep analysis data server 40 is incommunicable due to its own malfunction, the sleep analysis data server 40 may obtain the data supposed to be received from the sleep monitor 10 to the real-time data server 30 after returning to a normal operation.

It is noted that the sleep analysis data server 40 may send/receive the data during the communication failure to/from the real-time data server 30 in accordance with the predetermined notification from the sleep monitor 10.

The external terminal 50 is a communication terminal for displaying the state relating to user's sleep such as a PC and a mobile phone. The external terminal 50 has an external communication unit 51 as an interface for communication, a storage unit 52 for storing data received from the sleep analysis data server 40 and the real-time data server 30, and an analysis unit 53 for analyzing the data stored in the storage unit 52. The external terminal 50 accesses the real-time data server 30 and the sleep analysis data server 40 through the external communication unit 51, and presents the state relating to user's sleep to the user, an administrator and the like by means of a display unit such as a liquid crystal display (not shown), a sound unit such as a speaker (not shown) or the like. It is noted that since different information is required for each user and administrator, the system may configured so that the data to be received is preset in the storage unit 52 of the external terminal 50 by the user and the administrator, and only the necessary data is received from the real-time data server 30 and the data analysis server 40. This allows the user and the administrator to obtain the necessary information while the transmission processes of the real-time data server 30 and the sleep analysis data server 40 can be reduced.

In addition, when both of the real-time data server 30 and the sleep analysis data server 40 are incommunicable and the sleep monitor 10 sends the data supposed to be sent to both of the serves, the external terminal 50 may directly obtain and analyze the state relating to user's sleep from the data and present it to the user and the administrator. It is noted that when any one of the real-time data server 30 and the sleep analysis data server 40 is incommunicable and the sleep monitor 10 sends the data supposed to be sent to the incommunicable server, the external communication unit 17 may directly obtain and analyze the state relating to user's sleep from the data and present to the user and the administrator.

In addition, in case of communication failure, the external terminal 50 may identify the location (the sleeping monitor or the server) with the malfunction or failure and notify the communicable device (the sleep monitor or server) of it. For example, when the external terminal 50 cannot receive data from the real-time data server 30 for a predetermined time period, the external terminal 50 determines that the real-time data server 30 is incommunicable due to malfunction and the notifies sleep monitor 10 and the sleep analysis data server 40 of it. This allows the sleep monitor 10 and the data analysis server 40 to recognize the communication failure of the real-time data server 30 due to malfunction or failure. It is noted that, when the sleep monitor 10 and the data analysis server 40 are incommunicable due to malfunction or failure, the external terminal 50 may take the similar processing and notify the communicable device (the sleep monitor or server) of it.

### (Application Example 1: Monitoring a facility)

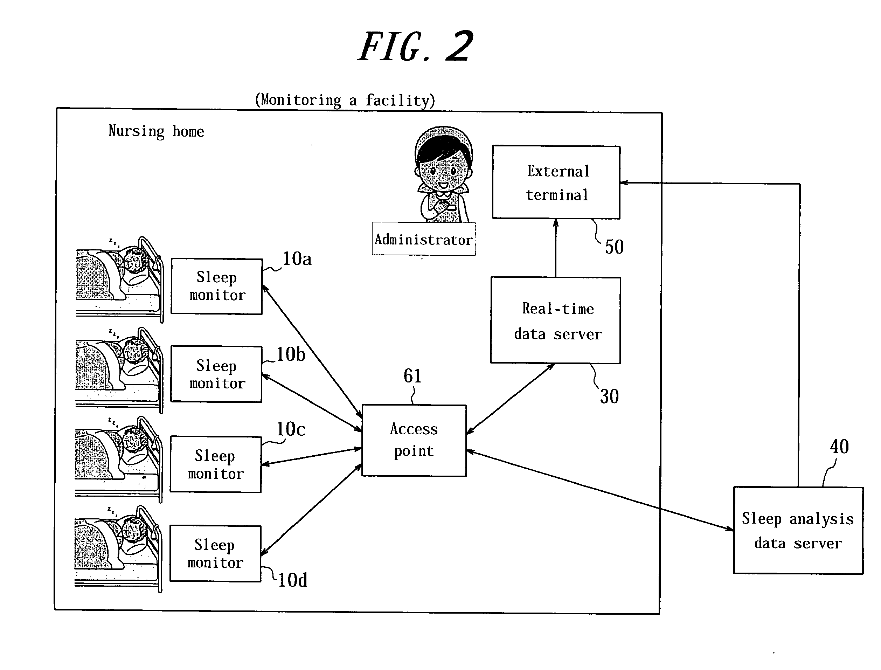

Figure 2 illustrates a first example of an application of the sleep management system according to the present invention. In Figure 2, a sleep management system is applied to monitoring a nursing home. Four sleep monitors 10a-10d, an access point 61 as a network connection unit, a real-time data server 30, and an external terminal 50 are installed inside of nursing home, and a sleep analysis data server 40 is installed outside of the nursing home. With said configuration, it is possible that the administrator checks the state of the resident such as the in-bed/out-of-bed information from the real-time data server 30 in a real time manner, and prevents the residents from nighttime wandering. It is also possible that the administrator confirms the analysis results of the residents from the sleep analysis data server 40 after each resident's sleep, and utilizes the results to improve residents' QOL (Quality Of Life). In this case, for example, the administrator may preset the external terminal 50 to receive only the out-of-bed information of the current epoch in a real time manner and only the sleep stage information in each epoch in the sleep analysis after the measurement, so that the transmission processes of the real-time data server 30 and the sleep analysis data server 40 can be reduced.

### (Application Example 2: Understanding employees' sleep state)

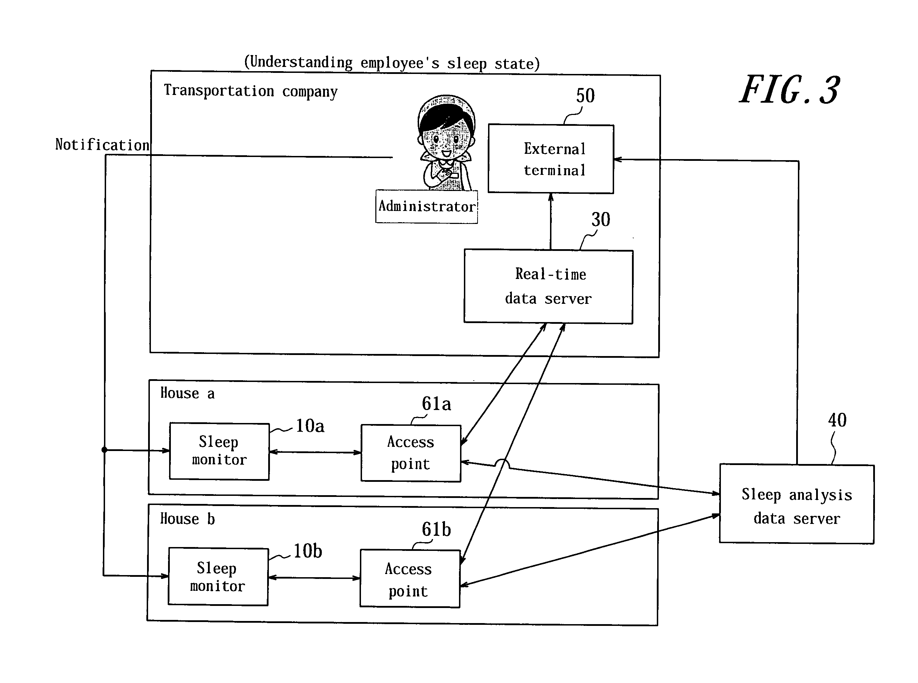

Figure 3 illustrate a second example of an application of the sleep management system according to the present invention. In Figure 3, the sleep management system is applied to understanding the sleep state of employees (drivers) of a transportation company. Sleep monitors 10a, 10b and access points 61 a, 61 b are installed in driver's houses a, b, respectively. A real-time data server 30 and an external terminal 50 are installed in the transportation company, and a sleep analysis data server 40 is installed in a facility other than the houses and the transport company. With this configuration, it is possible that the administrator checks the state of the drivers such as in-bed/out-of-bed information from the real-time date server 30 in a real time manner, and encourages the driver staying up late to go to sleep. It is also possible that the administrator confirms the analysis results of the drivers from the sleep analysis data server 40 after each resident's sleep, and recognizes the sleep durations and the health conditions of the drivers before driving.

In this way, according to the present embodiment, the sleep monitor 10 processes the biometric information in each epoch (unit interval), sends the data containing processing result of the biometric information in each epoch to the real-time data server 30 (first server), and sends the data of biometric information accumulated during the user's sleep and the data containing processing result of the biometric information in each epoch to the sleep analysis data server 40 (second server). As a result, it is possible to distinctly process a service requiring highly timely updates and a service requiring a sleep analysis to efficiently provide services with different natures. Also, the data such as heart rates, respiratory rates and the like in each epoch that imposes the heaviest load on the sleep analysis process is pre-calculated on the sleep monitor 10, so that the burden of the calculation of heart rates, respiration rates and the like on the sleep analysis data server 40 (second server) can be eliminated to reduce the load on the sleep analysis data server 40.

The sleep monitor 10 sends the data containing processing result of the biometric information in each epoch to the real-time data server 30 (first server), in every predetermined time period defining the epoch (unit interval). This allows the administrator to verify the latest data in each epoch through the external terminal 50 server and the real-time data server 30 in a real time manner.

The sleep monitor 10 sends the data accumulated up to the current time point and the data containing processing result of the biometric information in every epoch (second data) to the sleep analysis data server 40 at a certain time point during the sleep of the user. This can scatter the times for data transmission to resolve a disadvantage of data jam in, for example, the early morning, so that the burden on the sleep analysis data server 40 can be reduced.

The biometric information data preferably contains data of a plurality of channels with different amplification factors. This allows the sleep analysis data server 40 to utilize the data of the plurality of channels with different amplification factors, so that fluctuation caused by environmental influence can be identified to be able to conduct the sleep analysis regardless of the measurement environment.

The biometric information data preferably contains data of only one channel among data of a plurality of channels with different amplification factors. This can greatly reduce the amount of data sent to the sleep analysis data server 40 and thus reduce the processing load of the sleep analysis data server 40.

The external terminal 50 may select the data to be received from to the real-time data server 30 (first server) and the sleep analysis data server 40 (second server) for each user. This allows the administrator and the user to obtain necessary information, and allows the real-time data server 30 and the sleep analysis data server 40 to reduce the transmission processes.

When at least one of the real-time data server 30 (first server) and the sleep analysis data server 40 (second server) is incommunicable, the sleep monitor 10 notifies the external terminal 50 of the failure. Thus, in case of malfunction or failure, it is possible to notify the administrator and the user of the sleep management system of the necessary information in response to the malfunction.

In addition, in case of communication failure with any one of the real-time data server 30 (first server) and the sleep analysis data server 40 (second server), the sleep monitor 10 sends data supposed to be sent to the incommunicable server to the communicable server. Thus, when the incommunicable server recovers to a normal operation, the recovered server can obtain the data supposed to be received during the communication failure from the communicable server.

Further, when the incommunicable server recovers, the sleep monitor 10 sends a predetermined notice to at least one of the recovered and communicable servers so that the recovered server obtains the data supposed to be received during the communication failure from the communicable server.

The present invention has been discussed with reference to the various embodiments and the accompanying drawings. It should be appreciated that various variations and modifications of the present invention can be easily made by those skilled in the art. Therefore, it is noted that these variations and modifications are also within the scope of the present invention. For example, the features included in each part or each step can be rearranged with maintaining a logical consistency, and a plurality of function units and steps can be combined or divided.

### (Modified example: general monitor)

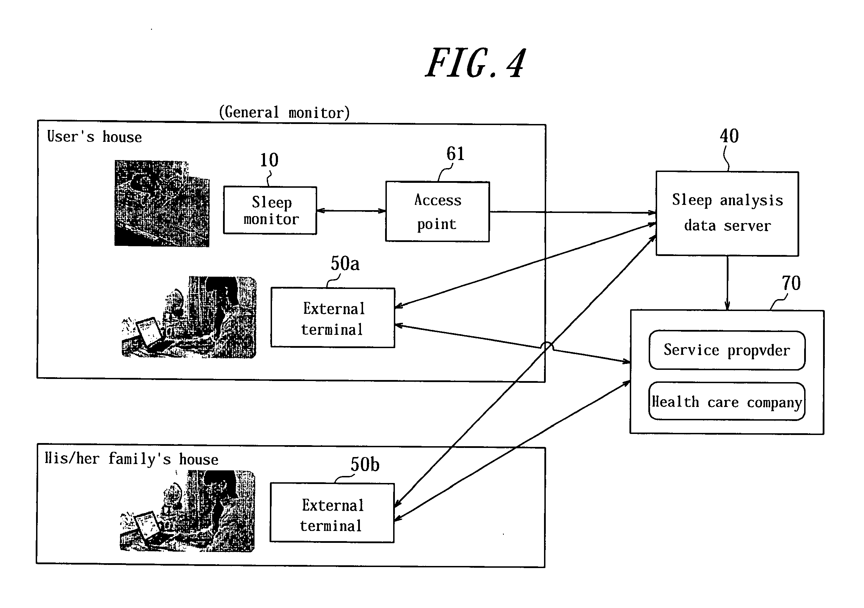

Figure 4 illustrates a modified example of the sleep management system according to the present invention. The modified example in Figure 4 is intended to a general monitoring service in cooperated with a third party. A sleep monitor 10, an access point 61, and an external terminal 50a are installed in a user's house, an external terminal 50b is installed in a his/her family's house, and a sleep data analysis server 40 and a server 70 of a third-party (a service provider, a health care company) are installed in a facility other than the user's house. The server 70 of the third party can obtain data from the sleep analysis data server 40 to provide a third-party proprietary service. Therefore, the user and his/her family not only can see the results of the sleep analysis from the sleep analysis data server 40, but also can enjoy the third-party proprietary service.

### [DESCRIPTION OF REFERENCE NUMERALS]

- 10: Sleep monitor
- 11: Matt
- 12: Sensor
- 13: Analog Circuits
- 14: Microcomputer
- 15: Storage unit
- 16: Display unit
- 17: External communication unit
- 20: Power source
- 30: Real-time data server (First server)
- 31: External communication unit
- 32: Storage unit
- 33: Analysis unit
- 40: Sleep analysis data server (Second server)
- 41: External communication unit
- 42: Storage unit
- 43: Analysis unit
- 50: External terminal
- 51: External communication unit
- 52: Storage unit
- 53: Analysis unit
- 60: Network connection equipment
- 61: Access points
- 70: Server

## Claims

1. A sleep management system comprising:
a sleep monitor for measuring biometric information relating to user's sleep,
an external terminal for displaying a state of said user's sleep,
a first server for storing first data received from said sleep monitor and providing the first data to said external terminal, and
a second server for analyzing said user's sleep on the basis of second data received from said sleep monitor and providing analysis result to said external terminal,
wherein said sleep monitor processes said biometric information of in each unit interval, sends a processing result of said biometric information in said each unit interval to said first server as said first data, and sends said biometric data accumulated during said user's sleep and said processing result of said biometric information in each said unit interval to said second sever as said second data.

2. The sleep managing system according to claim 1, wherein said sleep monitor sends said first data to said first server in every predetermined time period defining said unit interval.

3. The sleep management system according to claim 1 or 2, wherein, at a certain time point of said user's sleep, said sleep monitor sends said second data accumulated up to said time point to said second server.

4. The sleep management system according to any one of claims 1-3, wherein said biometric information data contains
data of a plurality of channels with different amplification factors or
data of only one channel among data of a plurality of channels with different amplification factors.

5. The sleep management system according to any one of claims 1-4, wherein said external terminal is capable of selecting the data to be received from said first and second servers for each said user.

6. The sleep management system according to any one of claims 1-5, wherein said sleep monitor, in case of communication failure with at least one of said first and second servers, notifies said external terminal of the failure.

7. The sleep management system according to any one of claims 1-6, wherein said sleep monitor, in case of communication failure with any one of said first and second servers, sends data supposed to be sent to the incommunicable server to the communicable server.

8. The sleep management system according to claim 7, wherein said sleep monitor, when said incommunicable server recovers from the failure, sends a predetermined notice to at least one of said recovered server and said communicable servers so that said recovered server obtains the data supposed to be received during the communication failure from said communicable server.

9. A sleep monitor comprises,
a sensor for measuring biometric information relating to user's sleep,
a microcomputer for processing said biometric information in each unit interval,
a storage unit for storing data of said biometric information and a processing result of said biometric information, and
an external communication unit for sending said data of said biometric information and said processing result of said biometric data,
wherein said external communication unit sends first data consisting of said processing result of said biometric information in each said unit interval, and second data consisting of said biometric data accumulated during said user's sleep and said processing result of biometric data in said each unit interval to first and second servers, respectively.

10. The sleep monitor according to claim 9, wherein said external communication unit sends said first data to said first server in every predetermined time period defining said unit interval.

11. The sleep monitor according to claim 9 or 10, wherein at a certain time point of said user's sleep, said external communication unit sends said second data accumulated up to said time point to said second server.

12. The sleep monitor according to any one of claims 9-11, wherein said biometric information data contains
data of a plurality of channels with different amplification factors or
data of only one channel among data of a plurality of channels with different amplification factors.

13. The sleep monitor according to any one of claims 9-12, wherein said external communication unit, in case of communication failure with at least one of said first and second servers, sends a notice of the failure.

14. The sleep monitor according to any one of claims 9-13, wherein said external communication unit, in case of communication failure with any one of said first and second servers, sends data supposed to be sent to the incommunicable server to the communicable server.

15. The sleep monitor according to claim 14, wherein said external communication unit, when said incommunicable server recovers from the failure, sends a predetermined notice to at least one of said recovered server and said communicable servers so that said recovered server obtains the data supposed to be received during the communication failure from said communicable server.
